Europäisches Patentamt

European Patent Office

Office européen des brevets

(11) Veröffentlichungsnummer: **0 145 798**
**B1**

(12) **EUROPÄISCHE PATENTSCHRIFT**

(45) Veröffentlichungstag der Patentschrift:
01.04.87

(21) Anmeldenummer: 83112408.6

(22) Anmeldetag: 09.12.83

(51) Int. Cl.⁴: **C 12 N 9/28**, C 12 N 1/20 //
(C12N9/28,
C12R1:125),(C12N1/20,
C12R1:125)

(54) Bacillus subtilis DSM 2704 und Verfahren zur Herstellung von Alpha-Amylase.

(43) Veröffentlichungstag der Anmeldung:
26.06.85 Patentblatt 85/26

(45) Bekanntmachung des Hinweises auf die Patenterteilung:
01.04.87 Patentblatt 87/14

(84) Benannte Vertragsstaaten:
AT BE CH DE FR GB IT LI NL SE

(56) Entgegenhaltungen:
DE - A - 2 044 513
DE - A - 2 218 783

MICROBIOLOGY ABSTRACTS SECT. A, Band 14, Nr. 11,
November 1979, Seite 9, Nr. 8298-A14, London, GB; B.
MARUO et al.: "Stepwise genetic transformation of
Bacillus subtilis with enhancement of productivity of
alpha-amylase"
CHEMICAL ABSTRACTS, Band 93, 1980, Seite 735, Nr.
68681y, Columbus, Ohio, USA
CHEMICAL ABSTRACTS, Band 86, 1977, Seite 375, Nr.
87645m, Columbus, Ohio, USA; WUSIH FERMENTATION
PLANT FOR ENZYMES et al: "Selection and industrial
application of a highly alpha-amylase-producing strain
209 from Bacillus subtilis BF-7658"
CHEMICAL ABSTRACTS, Band 78, 1973, Seite 323, Nr.
70185y, Columbus, Ohio, USA; J. SEKIGUCHI et al.:
"Regulation of alpha-amylase production in a Bacillus
subtilis Marburg strain. I. Isolation of mutants which
produce high levels of alpha-amylase and analysis of
their enzymes"

(73) Patentinhaber: **Miles Kali-Chemie GmbH & Co. KG
Biochemisches Werk, Postfach 1440 Grosse
Drakenburger Strasse 103, D-3070 Nienburg/Weser (DE)**

(72) Erfinder: **Lompe, Arved, Dr.rer.nat., Hoher Weg 2,
D-3070 Nienburg (DE)**

(74) Vertreter: **Lauer, Dieter, Dr., c/o Kali-Chemie AG
Postfach 220 Hans-Böckler-Allee 20,
D-3000 Hannover 1 (DE)**

**Beschreibung**

Alpha-Amylase ist eines der industriell bedeutsamsten Enzyme und wird in grossem Umfang beispielsweise in der Stärke-, Textil-, Lebensmittel-Industrie zur Hydrolyse von Stärke eingesetzt. Die industrielle Herstellung von Alpha-Amylase hat daher wirtschaftlich einiges Gewicht.

Zur Herstellung von Alpha-Amylase ist es allgemein bekannt, dass durch Fermentierung verschiedener Arten von Mikroorganismen der Gattung Bacillus, darunter Bacillus subtilis und dessen Varietäten, Alpha-Amylase in wirtschaftlich vertretbarer Ausbeute gewonnen werden kann. Besonders vorteilhaft ist es dabei, wenn schon in dem Fermentationsmedium möglichst hohe Konzentrationen an aktivem Enzym vorliegen. Es mangelt daher nicht an Bemühungen, um durch züchterische und verfahrenstechnische Massnahmen im Fermentationsmedium eine möglichst hohe Enzymkonzentration zu erzielen. So wird zum Beispiel von Yoneda et al. in der Zeitschrift «Applied and Environmental Microbiology»; Jahrgang 1980, Band 39, S. 274 ff und in dem Buch «Molecular Cloning and Gene Regulation in Bacilli», herausgegeben von Ganesan, Chang und Hoch, erschienen 1982 bei Academic Press, Inc., New York, ein Verfahren beschrieben, wie bei einem industriell genutzten Stamm von Bacillus subtilis durch gezielte Übertragung von Genfragmenten die Ausbeute der Alpha-Amylase in der Fermentationsbrühe von 130 bis 150 U/ml auf 25000 U/ml gesteigert werden konnte. Diese Steigerung stellt in diesem Fall eine ca. 170fache Verbesserung der Produktivität dar. Eine derartige Produktivität ist jedoch für eine industrielle Herstellung von Alpha-Amylase immer noch ungenügend.

Der Erfindung liegt daher die Aufgabe zugrunde, einen Mikroorganismus der Art Bacillus subtilis bereitzustellen bzw. ein Verfahren zur Herstellung von Alpha-Amylase unter Verwendung eines solchen Mikroorganismus zu entwickeln, bei dem eine wesentlich höhere Produktivität erzielbar ist.

Die Lösung dieser Aufgabe gelingt durch Bereitstellung des Mikroorganismus Bacillus subtilis DSM 2704 bzw. durch ein Verfahren zur Herstellung von Alpha-Amylase unter Verwendung dieses Mikroorganismus.

Der neue Mikroorganismus DSM 2704 wurde bei den in der industriellen Fermentation üblichen Kontrolluntersuchungen (Anfertigung von Ausstrichen von Proben der Fermentationsbrühe, Fermentierung von Kolonien solcher Ausstriche im Schüttelkolben, Anfertigung von Ausstrichen von Proben der Fermentationsbrühe aus den Schüttelkolben) als abweichende Kolonie auf dem Kontrollausstrich einer Probe aus dem Schüttelkolben beobachtet, durch systematisches Screening rein isoliert und als im morphologischen und physiologischen Verhalten sich vom ursprünglichen Mikroorganismus unterscheidende Mutante erkannt.

Der neue Mikroorganismus mit der internen Bezeichnung A4 ist unter der Hinterlegungsnummer DSM 2704 bei der Deutschen Sammlung von Mikroorganismen hinterlegt.

Die morphologischen und sotffwechselphysiologischen Eigenschaften des Mikroorganismus DSM 2704 wurden untersucht und zur Bestimmung mit den in «Bergey's Manual of determinative bacteriology» aufgeführten Eigenschaften verglichen. Im einzelnen wurden folgende Eigenschaften festgestellt:

| | |
|---|---|
| Katalasebildung: | positiv |
| Anaerobes Wachstum in Nähragar: | negativ |
| Gasbildung aus Glucose: | negativ |
| Gasbildung aus Xylose: | negativ |
| Gasbildung aus Mannitol: | negativ |
| Gasbildung aus Arabinose: | negativ |
| Gasbildung aus Trehalose: | negativ |
| pH auf Glucose: | 6,1 |
| pH auf Xylose: | 6,7 |
| pH auf Mannitol: | 6,1 |
| pH auf Arabinose: | 6,75 |
| pH auf Trehalose: | 6,8 |
| Verwertung von Propionat: | negativ |
| Verwertung von Citrat: | positiv |
| Wachstum bei 65°C: | negativ |
| Wachstum in Nährbouillon mit 5% NaCl: | positiv |
| Wachstum in Nährbouillon mit 7% NaCl: | positiv |
| Wachstum in Nährbouillon mit 10% NaCl: | positiv |
| Voges-Proskauer-Reaktion: | possitiv |
| pH in V.P. Bouillon: | 5,45 |
| Wachstum in 0,001% Lysozym: | negativ |
| Wachstum in 0,02% Na-azid: | negativ |
| Hydrolyse von Stärke: | positiv |
| Hydrolyse von Hippurat: | negativ |
| Zersetzung von Tyrosin: | negativ |
| Zersetzung von Casein: | positiv |
| Nitratreduktion: | positiv |
| Gelatineverflüssigung: | > 1 cm Ø |
| Eigelbreaktion: | negativ |

Die morphologischen Merkmale der Einzellen entsprechen den von Bacillus subtilis bekannten; ebenso ist die Koloniemorphologie mit der bekannter Stämme von Bacillus subtilis weitgehend identisch. Im direkten Vergleich mit den Ausgangskulturen fällt der Stamm A4 durch stark gekräuselte Kolonien mit unebener Oberfläche auf, die im Durchlicht bräunlich dunkel mit körniger Struktur erscheinen.

Unter Anwendung der im «Bergey's Manual of determinative bacteriology» angegebenen Bestimmungstabelle wurde grösstmögliche Ähnlichkeit festgestellt zu Bacillus subtilis (Ehrenberg) Cohn. Wir möchten daher diesen Stamm als zur Spezies Bacillus subtilis (Ehrenberg) Cohn gehörig bezeichnen.

Der neue Mikroorganismus DSM 2704 ist in der Produktivität von Alpha-Amylase allen bekannten Mikroorganismen der gleichen Gattung und Art weit überlegen. Die Konzentration an Alpha-Amylase in den Fermentationsmedien liegt konstant über 250000 U/ml und reicht zum Teil über 300000 U/ml hinaus. Besonders vorteilhaft an dem neuen Mikroorganismus ist insbesondere, dass er weniger Protease als die bekannten Mikroorganismen der Art Bacillus subtilis bildet. So lag die Konzentration an Protease im Fermentationsmedium in keinem Fall höher als 10 NU/100000 Amylaseeinheiten. Typischerweise liegt die Konzentration an Protease unter 8 NU/100000 Amylaseeinheiten.

Beansprucht werden auch alle natürlichen und künstlichen Mutanten des neuen Mikroorganismus Bacillus subtilis DSM 2704, insbesondere wenn sie bei Fermentation mindestens 200 000 U/ml, vorzugsweise mindestens 250 000 U/ml, Alpha-Amylase bilden.

Solche natürlichen und künstlichen Mutanten werden insbesondere dann beansprucht, wenn sie bei Fermentation höchstens 10 NU/100 000 Amylaseeinheiten, vorzugsweise höchsten 8 NU/100 000 Amylaseeinheiten, Protease bilden.

Die Fermentation des neuen Mikroorganismus Bacillus subtilis DSM 2704 bzw. die erfindungsgemässe Herstellung von Alpha-Amylase unter Verwendung des Stammes DSM 2704 kann sowohl in flüssigen Nährböden submers als auch auf festen Nährböden erfolgen, wobei die Herstellung in flüssiger Kultur im allgemeinen vorteilhafter ist. Die Fermentation erfolgt nach heute allgemein üblichen Verfahren. Die Herstellung der verwendbaren Nährböden erfolgt ebenso auf bekannte Weise. Die Nährböden enthalten assimilierbare Kohlenstoffquellen, assimilierbare Stickstoffquellen und andere, für das Wachstum des Mikroorganismus günstige oder notwendige Nähr- und Hilfsstoffe. Als Kohlenstoffquellen geeignet haben sich verschiedene Zucker und zuckerhaltige Stoffe erwiesen, wobei die Zucker in unterschiedlichen Polymerisationsstufen vorliegen können; als Beispiel seien genannt Stärke, Dextrine, Rohrzucker, Milchzucker, Maltose, Fructose, Glucose. Als Stickstoffquellen können sowohl anorganische als auch organische Stickstoffverbindungen sowohl einzeln als auch in Kombination verwendet werden. Als Beispiel seien genannt proteinhaltige Substanzen, wie z.B. Pepton aus Soja, Fleisch und Casein, Gelatine, Hefeeiweiss oder Hefeextrakt, Abfälle aus der Fleischerzeugung oder Tierkörperverwertung, Ammoniumsalze. Darüberhinaus kann der Zusatz von anorganischen Salzen, insbesondere von Alkali- und Erdalkali-Metall-Salzen und Phosphaten, von Vorteil sein, sowie die Zugabe von Spurenelementen, wie beispielsweise Fe, Mg, Mn, Co, Ni.

Die Fermentation wird bei pH-Werten zwischen 5 und 9, vorzugsweise zwischen 6 und 8, und einer Temperatur zwischen 25 und 50°C, vorzugsweise 33 und 45°C, durchgeführt. Die Fermentationsdauer beträgt zwischen 30 und 90 Stunden, vorzugsweise zwischen 50 und 70 Stunden.

Nachstehend sind die Beispiele für Fermentationen aufgeführt, die die Erfindung beschreiben, ohne dass diese auf die aufgeführten Beispiele beschränkt wird.

Die Bestimmung der Alpha-Amylase-Aktivität erfolgt nach einer von FUWA (1954) in der Zeitschrift «Journal of Biochemistry (Tokyo)», Jahrgang 1954, Band 41, Seite 583 ff beschriebenen Methode. Als Enzymsubstrat wurde hochgereinigte Amylose (Sigma Chemicals Co., Bestellnr. A 0512) verwendet. Alle anderen Reagentien waren Standardqualität «pro analysi».

Amyloselösung 0,2%:

200 mg Amylose werden in 4 ml 1 N NaOH gegeben und über Nacht im Kühlschrank stehengelassen. Nach Verdünnung auf ca. 80 ml wird mit 1 N Essigsäure neutralisiert und auf 100 ml aufgefüllt. Diese Lösung muss täglich frisch bereitet werden.

Reagenz A:

Lösung mit 0,2% Jod und 2% Kaliumjodid. Diese Lösung wurde täglich aus einer fünffach konzentrierten Stammlösung durch Verdünnen mit Wasser hergestellt.

Arbeitsweise:

500 µl 0,5 M Acetatpuffer und 500 µl enzymhaltige Lösung, gegebenenfalls mit Kalziumacetatlösung verdünnt, wurden mit 100 µl Amyloselösung (0,2%) gemischt und 30 Minuten im Wasserbad bei 37°C inkubiert.

Die Reaktion wurde durch Zugabe von 2000 µl 1 N Essigsäure gestoppt, das Gemisch 1 : 50 mit Reagenz A verdünnt und die Extinktion bei 700 nm und 10 mm Lichtweg gemessen. Eine Amylaseeinheit (U) entspricht einer 10%igen Reduktion des Jodstärkefarbkomplexes. Im Bereich von 1 bis ca. 5 Einheiten ist die Eichkurve linear.

Die Bestimmung der Protease-Aktivität erfolgt nach folgender Methode:

Caseinlösung:

0,2% Casein nach Hammarsten (Merck, Art.-Nr. 2242) in Puffer 1, durch Erwärmen auf 40°C für 45 Minuten, lösen.

Puffer 1:

10,3 g Borsäure, 46,18 g Dinatriumhydrogenphosphat × 7 $H_2O$, 60,7 Trinatriumcitrat × 2 $H_2O$ auf 2 l mit destilliertem Wasser auffüllen; pH = 8,0.

Puffer 2:

120 g Natriumacetat × 3 $H_2O$ und 160 ml Essigsäure auf 2 l mit destilliertem Wasser auffüllen; pH = 4,0.

Arbeitsweise:

Parallel zur Probe wird ein durch Erhitzen denaturierter Blindwert gemessen. 50 ml Caseinlösung (vortemperiert auf 40°C) + 1 ml Enzymlösung bzw. Blindwert (Enzymlösung so verdünnen, dass die Aktivität zwischen 0,07 und 0,11 NU/ml liegt) 35 Minuten bei 40°C inkubieren; Reaktion durch Zugabe von 25 ml Puffer 2 stoppen.

Nach 15 Minuten Verweildauer Lösung filtrieren (Blauband, Schleicher & Schüll Nr. 589[3]).

Im Filtrat wird Stickstoff nach dem Verfahren von Kjeldahl bestimmt; der Proteingehalt berechnet nach der Formel

$$\text{Protein} = \text{Stickstoff} \times 6{,}25.$$

1 Proteaseeinheit (NU) ist definiert als die Aktivität, die unter den Bedingungen des Tests 40% des Caseins hydrolysiert.

*Beispiel 1*

Zur Bereitung von 1 Liter Nährboden wurden 90 g Lactose, 30 g Sojamehl, 10 g Proteosepepton Oxoid Nr. L46 gemischt, à 50 ml in Erlenmeyerkolben abgefüllt, sterilisiert und mit Bacillus subtilis A 4 beimpft. Nach 70 Stunden Bebrütung auf einer Rundschüttelmaschine wurde scharf zentrifugiert und im Überstand die Aktivität bestimmt. Sie betrug im Mittel 300 000 U/ml; die Proteaseaktivität 21 NU/ml.

*Beispiel 2*

Zur Bereitung von 1 Liter Nährboden wurden 90 g Maltodextrin, 30 g Soja, 10 g Gelatine, 2,5 g Diammonphosphat gemischt, à 50 ml in Erlenmayer-Schikanenkolben abgefüllt, sterilisiert und wie in Beispiel 1 bebrütet. Die Ausbeute betrug im Mittel 280 000 U/ml; die Proteaseaktivität 22 NU/ml.

*Beispiel 3*

Das in Beispiel 1 aufgeführte Medium wurde in einem 2-Liter-Fermenter mit 650 ml Nutzvolumen mit einem Luftdurchsatz von 1,3 v/vm und einer Rührdrehzahl von $900^{-1}$ fermentiert. Die Ausbeute betrug nach 70 Stunden 300 000 U/ml; die Proteaseaktivität 24 NU/ml.

Die Aufarbeitung der erfindungsgemäss hergestellten Fermenterbrühe und die Gewinnung von Alpha-Amylase erfolgt nach allgemein bekannten Methoden und Verfahren.

**Patentansprüche für die Vertragsstaaten:**
BE, CH, LI, DE, FR, GB, IT, NL, SE

1. Bacillus subtilis DSM 2704 und dessen bei Fermentierung mindestens 200 000 U/ml, vorzugsweise mindestens 250 000 U/ml, Alpha-Amylase bildende natürliche und künstliche Mutanten.

2. Bacillus subtilis DSM 2704 und dessen bei Fermentierung mindestens 200 000 U/ml, vorzugsweise mindestens 250 000 U/ml, Alpha-Amylase und höchstens 10 NU/100 000 Amylaseeinheiten, vorzugsweise höchstens 8 NU/100 000 Amylaseeinheiten, Protease bildende natürliche und künstliche Mutanten.

3. Verfahren zur Herstellung von Alpha-Amylase durch Fermentierung nach an sich bekannten Verfahren von Mikroorganismen der Art Bacillus subtilis in einem Nährmedium mit für Mikroorganismen der Art Bacillus subtilis an sich bekannten Zusammensetzung und gegebenenfalls Abtrennung der Alpha-Amylase aus dem Fermentationsmedium, dadurch gekennzeichnet, dass man Mikroorganismen gemäss einem der Ansprüche 1 bis 2 fermentiert.

4. Verfahren nach Anspruch 3, dadurch gekennzeichnet, dass man bei einem pH von 5,0 bis 9,0, vorzugsweise bei einem pH von 6,0 bis 8,0, fermentiert.

5. Verfahren nach Anspruch 3 oder 4, dadurch gekennzeichnet, dass man bei 25 bis 50°C, vorzugsweise bei 33 bis 45°C, fermentiert.

6. Verfahren nach einem der Ansprüche 3 bis 5, dadurch gekennzeichnet, dass man 30 bis 90 Stunden, vorzugsweise 50 bis 70 Stunden fermentiert.

**Patentansprüche für den Vertragsstaat: AT**

1. Verfahren zur Herstellung von Alpha-Amylase durch Fermentierung nach an sich bekannten Verfahren von Mikroorganismen der Art Bacillus subtilis in einem Nährmedium mit für Mikroorganismen der Art Bacillus subtilis an sich bekannten Zusammensetzung und gegebenenfalls Abtrennung der Alpha-Amylase aus dem Fermentationsmedium, dadurch gekennzeichnet, dass man Bacillus subtilis DSM 2704 oder dessen bei Fermentierung mindestens 200 000 U/ml, vorzugsweise mindestens 250 000 U/ml, Alpha-Amylase bildende natürliche und künstliche Mutanten fermentiert.

2. Verfahren nach Anspruch 1, dadurch gekennzeichnet, dass man Bacillus subtilis DSM 2704 oder dessen bei Fermentierung mindestens 200 000 U/ml, vorzugsweise mindestens 250 000 U/ml, Alpha-Amylase und höchstens 10 NU/100 000 Amylaseeinheiten, vorzugsweise höchstens 8 NU/100 000 Amylaseeinheiten, Protease bildende natürliche und künstliche Mutanten fermentiert.

3. Verfahren nach Anspruch 1 oder 2, dadurch gekennzeichnet, dass man bei einem pH von 5,0 bis 9,0, vorzugsweise bei einem pH von 6,0 bis 8,0, fermentiert.

4. Verfahren nach einem der Ansprüche 1 bis 3, dadurch gekennzeichnet, dass man bei 25 bis 50°C, vorzugsweise bei 33 bis 45°C, fermentiert.

5. Verfahren nach einem der Ansprüche 1 bis 4, dadurch gekennzeichnet, dass man 30 bis 90 Stunden, vorzugsweise 50 bis 70 Stunden fermentiert.

**Claims for the Contracting States:**
BE, CH, LI, DE, FR, GB, IT, NL SE

1. Bacillus subtilis DSM 2704 and its natural and synthetic mutants forming on fermentation at least 200 000 U/ml, preferably at least 250 000 U/ml, alpha amylase.

2. Bacillus subtilis DSM 2704 and its natural and synthetic mutants forming on fermentation at least 200 000 U/ml, preferably at least 250 000 U/ml, alpha amylase and a maximum of 10 NU/100 000 amylase units, preferably a maximum of 8 NU/100 000 amylase units, protease.

3. Process for the preparation of alpha amylase by fermentation according to methods known per se for micro-organisms of the bacillus subtilis type in a nutrient medium with a composition known per se for micro-organisms of the bacillus subtilis type, and, if required, a separation of the alpha amylase from the fermentation medium, characterized in that micro-organisms according to one of claims 1 to 2 are fermented.

4. Process according to claim 3, characterized in that fermentation is carried out at a pH 5.0 to 9.0, preferably at a pH of 6.0 to 8.0.

5. Process according to claim 3 or 4, characterized in that fermentation is carried out at 25 to 50°C, preferably at 33 to 45°C.

6. Process according to one of claims 3 to 5, characterized in that fermentation is carried out for 30 to 90 hours, preferably 50 to 70 hours.

**Claims for the Contracting State: AT**

1. Process for the preparation of alpha amylase by fermentation according to methods known per se for micro-organisms of the bacillus subtilis type in a nutrient medium with a composition known per se for micro-organisms of the bacillus subtilis type, and, if required, a separation of the alpha amylase

from the fermentation medium, characterized in that bacillus subtilis DSM 2704 or its natural and synthetic mutants forming on fermentation at least 200 000 U/ml, preferably at least 250 000 U/ml, alpha amylase is or are fermented.

2. Process according to claim 1, characterized in that bacillus subtilis DSM 2704 or its natural and synthetic mutants forming on fermentation at least 200 000 U/ml, preferably at least 250 000 U/ml, alpha amylase and a maximum of 10 NU/100 000 amylase units, preferably a maximum of 8 NU/100 000 amylase units, protease is or are fermented.

3. Process according to claim 1 or 2, characterized in that fermentation is carried out at a pH 5.0 to 9.0 preferably at a pH of 6.0 to 8.0.

4. Process according to one of claims 1 to 3, characterized in that fermentation is carried out at 25 to 50°C, preferably at 33 to 45°C.

5. Process according to one of claims 1 to 4, characterized in that fermentation is carried out for 30 to 90 hours, preferably 50 to 70 hours.

**Revendications pour les Etats contractants:**
**BE, CH, LI, DE, FR, GB, IT, NL, SE**

1. Bacillus Subtilis DSM 2704 et ses mutants naturels et artificiels donnant naissance par fermentation à au moins 200 000 U/ml, de préférence à au moins 250 000 U/ml d'alpha-amylase.

2. Bacillus Subtilis DSM 2704 et ses mutants naturels et artificiels donnant naissance par fermentation à au moins 200 000 U/ml, de préférence à au moins 250 000 U/ml d'alpha-amylase et à au plus 10 NU/100 000 unités d'amylase, de préférence à au plus 8 NU/100 000 unités d'amylase, de protéase.

3. Procédé de préparation d'alpha-amylase par fermentation selon des procédés connus en eux-même de microorganismes de l'espèce Bacillus Subtilis dans un milieu de culture de composition connue en elle-même pour des microorganismes de l'espèce Bacillus Subtilis et éventuellement séparation de l'alpha-amylase du milieu de fermentation, caractérisé en ce que l'on fait fermenter des microorganismes selon l'une des revendications 1 et 2.

4. Procédé selon la revendication 3, caractérisé en ce que l'on effectue la fermentation à un pH de 5,0 à 9,0 de préférence à un pH de 6,0 à 8,0.

5. Procédé selon la revendication 3, caractérisé en ce que l'on effectue la fermentation à 25 à 50°C, de préférence 33 à 45°C.

6. Procédé selon l'une des revendications 3 à 5, caractérisé en ce que l'on effectue la fermentation pendant 30 à 90 heures, de préférence 50 à 70 heures.

**Revendications pour l'Etat contractant: AT**

1. Procédé de préparation d'alpha-amylase par fermentation selon des procédés connus en eux-même de microorganismes de l'espèce Bacillus Subtilis dans un milieu de culture de composition connue en elle-même pour des microorganismes de l'espèce Bacillus Subtilis et éventuellement séparation de l'alpha-amylase du milieu de fermentation, caractérisé en ce que l'on fait fermenter du Bacillus Subtilis DSM 2704 ou ses mutants naturels ou artificiels donnant naissance par fermentation à au moins 200 000 U/ml, de préférence à au moins 250 000 U/ml d'alpha-amylase.

2. Procédé selon la revendication 1, caractérisé en ce que l'on fait fermenter du Bacillus Subtilis DSM 2704 ou ses mutants naturels ou artificiels donnant naissance par fermentation à au moins 200 000 U/ml, de préférence à au moins 250 000 U/ml d'alpha-amylase et à au plus 10 NU/100 000 unités d'amylase, de préférence à au plus 8 NU/100 000 unités d'amylase, de protéase.

3. Procédé selon l'une des revendications 1 et 2, caractérisé en ce que l'on effectue la fermentation à un pH de 5,0 à 9,0, de préférence à un pH de 6,0 à 8,0.

4. Procédé selon l'une des revendications 1 à 3, caractérisé en ce que l'on effectue la fermentation à 25 à 50°C, de préférence à 33 à 45°C.

5. Procédé selon l'une des revendications 1 à 4, caractérisé en ce que l'on effectue la fermentation pendant 30 à 90 heures, de préférence pendant 50 à 70 heures.